# EUROPÄISCHE PATENTSCHRIFT

(11) **EP 3 551 569 B1**
(45) Veröffentlichungstag und Bekanntmachung des Hinweises auf die Patenterteilung: **19.10.2022**
(21) Anmeldenummer: 17811602.6
(22) Anmeldetag: 11.12.2017
(51) Int. Cl.: B67C 3/00, A61L 2/07

(54) **FÜLLVORRICHTUNG ZUM ABFÜLLEN EINES FÜLLPRODUKTS IN EINEN ZU BEFÜLLENDEN BEHÄLTER IN EINER FÜLLPRODUKTABFÜLLANLAGE**
FILLING DEVICE FOR FILLING A FILLABLE CONTAINER WITH A FILLER PRODUCT, IN A FILLER PRODUCT FILLING INSTALLATION
DISPOSITIF DE REMPLISSAGE DESTINÉ À REMPLIR UN CONTENANT À REMPLIR D'UN PRODUIT DE REMPLISSAGE DANS UNE INSTALLATION DE REMPLISSAGE EN PRODUIT DE REMPLISSAGE

(30) Priorität: 09.12.2016 DE 102016123965
(43) Veröffentlichungstag der Anmeldung: 16.10.2019
(73) Patentinhaber: Krones AG, 93073 Neutraubling (DE)
(72) Erfinder: BAUMGARTNER, Sebastian, 93073 Neutraubling (DE); WEBER, Thomas, 93073 Neutraubling (DE); SOELLNER, Juergen, 93073 Neutraubling (DE); KNITL, Thomas, 93073 Neutraubling (DE); SCHAFACZEK, Bernd, 93073 Neutraubling (DE)
(74) Vertreter: Nordmeyer, Philipp Werner
(86) Internationale Anmeldenummer: PCT/EP2017/082197
(87) Internationale Veröffentlichungsnummer: WO 2018/104551

(56) Entgegenhaltungen:
- EP-A1- 0 590 505
- EP-A1- 3 015 418
- EP-A2- 2 409 948
- JP-A- 2004 001 850
- US-A- 3 517 688
- US-A1- 2010 276 028

## Beschreibung

### Technisches Gebiet

Die vorliegende Erfindung betrifft eine Füllvorrichtung zum Abfüllen eines Füllprodukts in einen zu befüllenden Behälter in einer Füllproduktabfüllanlage und ein Verfahren zum Behandeln einer Füllvorrichtung, bevorzugt zum Reinigen und/oder Sterilisieren der Füllvorrichtung.

### Hintergrund der Erfindung

Füllvorrichtung zum Abfüllen eines Füllprodukts in einen zu befüllenden Behälter und Verfahren zum Behandeln, insbesondere zum Reinigen und/oder Sterilisieren, solcher Füllvorrichtungen sind aus dem Stand der Technik bereits bekannt.

Es ist bekannt, in Füllproduktabfüllanlagen Getränke in Behälter oder Dosen abzufüllen. Hierfür sind Füllorgane vorgesehen, welche dazu dienen, das Füllprodukt von einem Füllproduktreservoir über einen Füllproduktauslauf, der mit der jeweiligen Mündung des zu befüllenden Behälters in Übereinstimmung gebracht wird, mit dem Füllprodukt zu befüllen und so das in den Behälter einzufüllende Füllvolumen zu steuern. Um eine korrekte Dosierung des Füllprodukts in den Behälter zu erreichen, umfasst die Füllvorrichtung üblicherweise mindestens ein Füllventil, welches über einen Aktuator ansteuerbar ist. Mittels des Aktuators kann das Füllventil zu einem bestimmten Zeitpunkt geöffnet und zu einem weiteren Zeitpunkt geschlossen werden, um die vorgegebene Füllmenge des Füllprodukts in den Behälter einfließen zu lassen. Die Füllventile sind üblicherweise an einer Transportvorrichtung der Füllvorrichtung angeordnet, welche in der Regel in Form eines Rundläuferkarussells ausgebildet ist, um einen kontinuierlichen Abfüllbetrieb zu ermöglichen.

Zur Reinigung und Sterilisierung der bekannten Füllvorrichtungen sind unterschiedliche Verfahren bekannt. Als besonders vorteilhaft hat sich dabei beispielsweise das so genannte Cleaning-In-Place (CIP)-Verfahren erwiesen, bei welchem die Füllventile zur Reinigung nicht ausgebaut werden müssen, sondern im eingebauten Zustand mit dem Reinigungsmedium oder dem Sterilisierungsmedium durchspült beziehungsweise bedämpft werden. Dabei ist es beispielsweise bekannt, die Füllvorrichtung und insbesondere die Oberflächen, welche mit dem Füllprodukt in Berührung kommen, mittels Heißdampf thermisch zu sterilisieren. Hierzu werden die Füllventile mit Heißdampf beaufschlagt und auf dieses Weise auf die Sterilisierungstemperatur gebracht und auf dieser gehalten. Die daraus resultierende Temperatur der Füllventile wird über eine bestimmte Zeit aufrechterhalten und überwacht, um eine vorgegebene Sterilisationswirkung zu erzielen, wie beispielsweise aus der DE 10 2010 031 873 A1 oder der DE 10 2013113621 A1 bekannt ist.

Das Verfahren für die Sterilisierung wird auch als Sterilization-In-Place (SIP) bezeichnet.

Zur Reduzierung des Dampfverbrauchs bei gleichzeitiger Aufrechterhaltung des Drucks, zur Ableitung von Kondensat und zur besseren Detektion des Dampfdurchsatzes wird während der thermischen Sterilisation eine Blende mit einer kleinen Öffnung vor den Auslauf des Füllventils geschwenkt und dichtend mit diesem verbunden. Durch die kleine Öffnung der Blende hindurch tritt dann ein Dampfstrahl aus, dessen dynamischer Druck mittels eines unterhalb der Blende angeordneten Drucksensors detektiert wird. Die Temperatur wird dabei indirekt durch das Erfassen des vorgelagerten Sattdampfdruckes und des Dampfdurchsatzes bzw. Dampfdruckes im Füllventil über den somit erfassten Differenzdruck pro Füllventil ermittelt. Üblicherweise ist bei Rotationsmaschinen der Drucksensor statisch unterhalb der Höhe der Blende verbaut. Der Druck des aus der Blende austretenden Dampfstrahls wird dabei erfasst, wenn das jeweilige Füllventil über den Drucksensor bewegt wird.

Insbesondere wenn für hygienische Anwendungen das Abfüllen des Füllprodukts in einem Abfüllraum beziehungsweise Reinraum der Füllvorrichtung erfolgt, in welchem im Vergleich zur Umgebung eine reduzierte mikrobielle Belastung und/oder ein Überdruck vorliegt und mithin der Sterilitätsgrad gegenüber der Umgebung erhöht ist, ist das Vorsehen von zusätzlichen Bauteilen und Komponenten, wie der Blende, einer Hub- und Verschwenkvorrichtung für die Blende oder den Drucksensor aus hygienischer Sicht nachteilig, da die zusätzlich im Reinraum verbauten Bauteile zu einem erhöhten Reinigungsaufwand führen.

Eine weitere Möglichkeit der Temperaturüberwachung besteht nach dem Stand der Technik darin, jedes einzelne Füllventil mit einem Thermoelement zu überwachen, welches in der Regel in dem Füllventil integriert oder an der Blende angeordnet ist. Dies erfordert jedoch ein Thermoelement pro Füllventil beziehungsweise pro Blende sowie eine entsprechend aufwendige Verdrahtung, welche bei Rundläufermaschinen im drehenden Teil verbaut ist.

Die JP 2004 001850 A beschreibt eine Vorrichtung mit einem Infrarotthermometer zur Überwachung der Reinigung oder Sterilisierung eines Füllorgans. Die US 3,517,688 A beschreibt ein aseptisches Füllventil. Die EP 3 015 418 A1 beschreibt ein Verfahren und eine Vorrichtung zur Sterilisierung der Leitung eines Getränkeversorgungssystems.

### Darstellung der Erfindung

Ausgehend von dem bekannten Stand der Technik ist es eine Aufgabe der vorliegenden Erfindung, eine verbesserte Füllvorrichtung zum Abfüllen eines Füllprodukts in einen zu befüllenden Behälter in einer Füllproduktabfüllanlage, sowie ein verbessertes Verfahren zur Behandlung einer Füllvorrichtung bereitzustellen.

Die Aufgabe wird durch eine Füllvorrichtung zum Abfüllen eines Füllprodukts in einen zu befüllenden Behälter in einer Füllproduktabfüllanlage mit den Merkmalen des Anspruchs 1 gelöst. Vorteilhafte Weiterbildungen ergeben sich aus den Unteransprüchen sowie der vorliegenden Beschreibung und den Figuren.

Entsprechend wird eine Füllvorrichtung zum Abfüllen eines Füllprodukts in einen zu befüllenden Behälter in einer Füllproduktabfüllanlage vorgeschlagen, umfassend mindestens ein Füllventil zum Dosieren des Füllprodukts in den zu befüllenden Behälter sowie eine Sterilisierungsvorrichtung zum Sterilisieren zumindest eines Teils des mindestens einen Füllventils. Erfindungsgemäß ist ein berührungsloser Temperatursensor zum berührungslosen Erfassen der Temperatur des Füllventils vorgesehen.

Dadurch, dass ein berührungsloser Temperatursensor zum berührungslosen Erfassen der Temperatur des Füllventils vorgesehen ist, kann auf eine Blende, wie sie im Stand der Technik notwendig ist, verzichtet werden, die Füllvorrichtung mithin einen deutlich einfacheren Aufbau aufweisen und kompakter ausgeführt sein. Zudem kann hierdurch der Reinigungsaufwand vermindert werden, da insgesamt weniger Bauteile gereinigt werden müssen und insbesondere eine Reinigung von sich relativ zueinander bewegenden Teilen und deren Dichtungen, insbesondere der Blende und deren Bewegungsvorrichtung, entfällt.

Durch die Überwachung der Temperatur des Füllventils besonders beim Sterilisierungsvorgang kann sichergestellt werden, dass zumindest das Füllventil für eine vorgegebene Zeit mit der vorgegebenen Temperatur beaufschlagt wird, um eine vollständige Sterilisierung zu erreichen.

Durch die berührungslose Temperaturmessung wird darüber hinaus auch eine Kontaminierung des Füllventils und damit der füllproduktführenden Wege der Füllvorrichtung durch einen berührenden Temperatursensor vermieden. Entsprechend kann die Vorrichtung insgesamt kompakter und hygienisch vorteilhafter aufgebaut werden.

Die Sterilisierungsvorrichtung verwendet bevorzugt Heißdampf zum Sterilisieren. Es sind jedoch auch alle anderen bekannten Sterilisierungsverfahren denkbar, bei denen es darauf ankommt, dass die zu sterilisierende Oberfläche über einen vorgegebenen Zeitraum hinweg auf einer vorgegebenen Temperatur gehalten wird. Die berührungslose Temperaturmessung kann auch bei diesen Sterilisierungsverfahren vorteilhaft eingesetzt werden.

Die berührungslose Messung der Temperatur kann bevorzugt an einer Außenseite des Füllventils, bevorzugt im Bereich eines Ventilsitzes des Füllventils, durchgeführt werden, so dass die Temperatur des Füllventils berührungslos direkt erfasst und überwacht werden kann. Dabei kann davon ausgegangen werden, dass bei einer Durchleitung beispielsweise von Heißdampf durch die füllproduktberührten Wege bei einer Temperaturmessung am Füllventil auch die stromaufwärts des Füllventils liegenden Wege zumindest die gleiche Temperatur aufweisen, wie das Füllventil. Damit kann über die Temperaturmessung des Füllventils eine Sterilisierung aller füllproduktberührten Wege dokumentiert und sichergestellt werden.

Des Weiteren ist hierdurch eine höhere Prozesssicherheit ermöglicht, da es bei berührungslos messenden Sensoren nicht wie bei berührend messenden Sensoren zu einem Ablösen des Sensors von der zu messenden Fläche oder zu einem Abrieb oder einer Deformation des Sensors kommen kann.

Der berührungslose Temperatursensor ist bevorzugt auf eine Außenseite beziehungsweise Außenfläche des mindestens einen Füllventils gerichtet. Bevorzugt erfasst der berührungslose Temperatursensor die Temperatur des Füllventils dabei im Bereich des Ventilsitzes. So kann die Temperatur des Füllventils einfach und präzise erfasst werden.

Das Behandlungsmedium für die Sterilisierung kann bevorzugt Dampf, besonders bevorzugt Heißdampf, aufweisen. Alternativ und/oder zusätzlich kann das Behandlungsmedium auch Wasserstoffperoxid oder Peressigsäure, bevorzugt in Kombination mit Alkoholen, besonders bevorzugt Ethanol, aufweisen.

Um die Temperatur des mindestens einen Füllventils besonders exakt und zuverlässig ermitteln zu können, ist in einer weiteren bevorzugten Ausführungsform der zumindest eine berührungslose Temperatursensor in Form eines Pyrometers und/oder einer Infrarotkamera ausgebildet. Insbesondere, wenn der berührungslose Temperatursensor als Infrarotkamera ausgebildet ist, kann dieser die Temperatur mehrerer Füllventile gleichzeitig erfassen, so dass ein nochmals vereinfachter Aufbau der Füllvorrichtung und eine nochmals erhöhte Prozesssicherheit erzielt wird. Durch eine entsprechende Auswertungssoftware kann damit für jedes individuelle Füllventil die Temperatur ermittelt werden.

Erfindungsgemäß ist ein Reinraum vorgesehen, und zumindest ein Füllproduktauslass des Füllventils ist in dem Reinraum angeordnet, wobei der zumindest eine berührungslose Temperatursensor außerhalb des Reinraums so angeordnet ist, dass er die Temperatur des Füllventils im Reinraum erfassen kann.

Zum einen ist der berührungslose Temperatursensor dadurch frei zugänglich. Mit anderen Worten muss bei einem Zugriff auf den Temperatursensor, beispielsweise durch Bedienungs- oder Wartungspersonal, nicht in den Reinraum eingegriffen werden, so dieser intakt bleiben kann. Eine Wartung des Temperatursensors ist somit trotz des Abfüllens des Füllprodukts im Hygienebetrieb besonders einfach. Weiterhin ist im Inneren des Reinraums die Gefahr des Entstehens von mikrobiellen Verunreinigungen und somit der Reinigungsaufwand vermindert. Da die Sensorik zu Erfassen der Temperatur des Ventilsitzes außerhalb des Reinraums angeordnet ist, wird weiterhin verhindert, dass sie durch Spritzer des Füllprodukts verunreinigt wird, was andernfalls wiederum zu Beeinträchtigungen im Messergebnis des Temperatursensors führen könnte.

Um ein besonders effizientes Abfüllen des Füllprodukts zu ermöglichen, kann das mindestens eine Füllventil an einer Transportvorrichtung, bevorzugt einem Rundläuferkarussell, angeordnet sein.

In einer weiteren bevorzugten Ausführungsform ist eine geringere Anzahl berührungsloser Temperatursensoren als Füllventile vorgesehen und bevorzugt ist ein einziger berührungsloser Temperatursensor zur berührungslosen Erfassung der Temperatur aller Füllventile vorgesehen, wobei Füllventile relativ zu den berührungslosen Temperatursensoren bewegbar sind, wobei bevorzugt die Temperatur des Füllventils bei einem Vorbeibewegen des mindestens einen Füllventils an dem zumindest einen berührungslosen Temperatursensor erfasst werden kann. Dadurch, dass eine geringere Anzahl an Temperatursensoren als Füllventile vorgesehen ist, kann der Aufwand für die Temperaturmessung reduziert werden. Dies ist besonders augenfällig bei der Verwendung nur eines einzigen Temperatursensors für die gesamte Füllvorrichtung. Da die Füllventile an dem Temperatursensor vorbeibewegt werden, kann dennoch eine zuverlässige Messung der Temperatur aller Füllventile vorgenommen werden.

Der berührungslose Temperatursensor ist dabei bevorzugt an einer festen Position angeordnet, so dass er auch mit einer Vorrichtung zum Verarbeiten des erfassten Temperaturwerts, bevorzugt einer Steuerungs- und/oder Regelungseinheit, besonders einfach verbunden werden kann und insbesondere die Übergabe des jeweiligen Signals von einem drehenden Teil an ein stehendes Teil nicht notwendig ist. Zudem ist es insbesondere bei Füllvorrichtungen in Rundläuferbauweise möglich, eine Vielzahl von an dem Rundläuferkarussell angeordneten Füllvorrichtungen zu überwachen, wobei die Temperatur der einzelnen Füllventile jeweils ermittelt werden kann, wenn das jeweilige Füllventil an dem Temperatursensor vorbeibewegt wird beziehungsweise durch dessen Messbereich bewegt wird. Die Füllvorrichtung kann dann besonders einfach aufgebaut sein. Zudem ist hierdurch die Störanfälligkeit sowie die Komplexität der Füllvorrichtung reduziert, da lediglich ein Sensor vorzusehen ist, mit dem die Temperatur einer Vielzahl von Füllventilen überwacht werden kann.

In einer weiten bevorzugten Ausführungsform ist eine Mehrzahl von Füllventilen vorgesehen, wobei die Mehrzahl von Füllventilen bevorzugt an einer Transportvorrichtung angeordnet ist, und jedem der Füllventile jeweils ein berührungsloser Temperatursensor zugeordnet ist. Dadurch kann die Temperatur jedes Füllventils besonders exakt erfasst werden. Zudem ist ein permanentes Erfassen und mithin ein permanentes Überwachen der Temperatur des Füllventils ermöglicht, so dass ein Reinigungs- und/oder Sterilisierungsvorgang besonders sicher erfolgen kann. Dadurch kann auch sichergestellt werden, dass die Temperatur eines jeden Füllventils zu jederzeit erfassbar ist und mithin ein besonders präzises Überwachen und Steuern/Regeln des Behandlungsvorgangs erzielbar ist.

Ein besonders effizientes Abfüllen des Füllprodukts kann erzielt werden, wenn eine Mehrzahl von Füllventilen vorgesehen ist, wobei die Mehrzahl von Füllventilen bevorzugt an der Transportvorrichtung angeordnet ist. So können mehrere zu befüllende Behälter im Wesentlichen zeitgleich durch die Mehrzahl von Füllventilen befüllt werden.

In einer weiteren bevorzugten Ausführungsform ist eine mit dem zumindest einen berührungslosen Temperatursensor verbundene Steuerungs- und/oder Regelungseinheit des Behandlungsvorgangs vorgesehen, welche die Behandlung des mindestens einen Füllventils anhand des durch den zumindest einen berührungslosen Temperatursensor erfassten Temperaturwerts des mindestens einen Füllventils steuert/regelt. Dadurch kann ein besonders effizientes und ressourcenschonendes Sterilisieren erzielt werden, da die zuzuführende Dampfmenge anhand der Temperatur genau geregelt werden kann und entsprechend ein übermäßiger Dampfverbrauch vermieden werden kann.

Wenn die Steuerungs- und/oder Regelungseinheit, wie in einer bevorzugten weiteren Ausführungsform, derart ausgebildet ist, dass das zumindest eine Füllventil während des Behandlungsvorgangs geöffnet wird, wenn der durch den berührungslosen Temperatursensor erfasste Temperaturwert des Füllventils unter einen vorgegebenen Temperaturwert fällt, kann auch Behandlungsmedium eingespart werden. Zum anderen kann dann durch den Temperaturabfall etwaig entstehendes Kondensat ausgelassen, bevorzugt ausgeblasen, und das Innere des Füllventils mitsamt dem Auslass des Füllventils und der Innenseite des Bodenstücks des Füllventils mit einer etwaig am Auslass des Füllventils angeordneten Zentrierglocke so lange mit Behandlungsmedium bespült beziehungsweise bedämpft werden, bis die Temperatur des Füllventils wieder über den vorgegebenen Temperaturwert steigt beziehungsweise das Kondensat ausgelassen ist.

Besonders bevorzugt erfolgt das Öffnen und Schließen des Füllventils alternierend. Dabei wird das Füllventil je nach erfasstem Temperaturwert geöffnet oder wieder geschlossen. Mit anderen Worten wird das Füllventil temperaturabhängig getaktet. So wird folglich nur dann Behandlungsmedium über das geöffnete Füllventil ausgelassen, wenn es zum Aufheizen und/oder Ablassen von Kondensat erforderlich ist.

Die oben gestellte Aufgabe wird weiterhin durch ein Verfahren zum Sterilisieren zumindest eines Teils mindestens eines Füllvorrichtung einer Füllproduktabfüllanlage mit den Merkmalen des Anspruchs 7 gelöst. Vorteilhafte Weiterbildungen des Verfahrens ergeben sich aus den Unteransprüchen sowie der vorliegenden Beschreibung und den Figuren.

Entsprechend wird ein Verfahren zum Sterilisieren zumindest eines Teils mindestens eines Füllventils in einer Füllproduktabfüllanlage vorgeschlagen, umfassend das Beaufschlagen des zumindest einen Füllventils mit einem Sterilisationsmedium. Erfindungsgemäß wird die Temperatur des zumindest einen Füllventils berührungslos erfasst.

Dadurch, dass die Temperatur des zumindest einen Füllventils berührungslos erfasst wird, können die bereits hinsichtlich der Füllvorrichtung erwähnten Vorteile erzielt werden.

Erfindungsgemäß ist ein Reinraum vorgesehen, und zumindest ein Füllproduktauslass des Füllventils ist in dem Reinraum angeordnet, und die Temperatur des mindestens einen Füllventils wird außerhalb des Reinraums erfasst.

Damit kann eine vorteilhafte hygienische Ausgestaltung der Anlage und des Verfahrens erreicht werden.

Ferner kann es vorteilhaft sein, wenn die die Sterilisierung des mindestens einen Füllventils anhand des berührungslos erfassten Temperaturwerts des mindestens einen Füllventils gesteuert/geregelt wird.

Damit kann ein effizientes und sicheres Durchführen des Sterilisationsvorgangs erreicht werden, welcher auch die Betriebskosten reduzieren kann, da der Einsatz des Sterilisationsmittels und insbesondere des Heißdampfs in der gerade notwendigen Menge durchgeführt werden kann.

In einer bevorzugten Weiterführung wird das zumindest eine Füllventil während der Sterilisierung geöffnet, wenn der berührungslos erfasste Temperaturwert des Füllventils unter einen vorgegebenen Temperaturwert fällt.

Dadurch kann einerseits Behandlungsmedium eingespart werden. Zum anderen wird dann durch den Temperaturabfall etwaige entstehendes Kondensat ausgelassen und das Innere des Füllventils mitsamt dem Auslass des Füllventils und der Innenseite des Bodenstücks des Füllventils mit einer etwaig am Auslass des Füllventils angeordneten Zentrierglocke so lange mit Behandlungsmedium bespült beziehungsweise bedämpft, bis die Temperatur des Füllventils wieder über den vorgegebenen Temperaturwert steigt beziehungsweise das Kondensat ausgelassen ist. Bevorzugt erfolgt das Öffnen und Schließen des Füllventils mehrmalig abwechselnd. Dabei wird das Füllventil temperaturabhängig getaktet. Mit anderen Worten wird das Füllventil geöffnet, wenn der erfasste Temperaturwert unterhalb des vorgegebenen Temperaturwerts fällt und wieder geschlossen, wenn der Temperaturwert wieder oberhalb des vorgegebenen Temperaturwerts liegt. So wird folglich auch nur dann Behandlungsmedium über das geöffnete Füllventil ausgelassen, wenn es zum Aufheizen und/oder Ablassen von Kondensat erforderlich ist.

Um die Steuerung/Regelung zu vereinfachen, kann alternativ bei einem Unterschreiten des vorgegeben Temperaturwerts das Füllventil auch lediglich für ein vorgegebenes Zeitintervall geöffnet werden.

### Kurze Beschreibung der Figuren

Bevorzugte weitere Ausführungsformen der Erfindung werden durch die nachfolgende Beschreibung der Figuren näher erläutert. Dabei zeigen:
- Figur 1: schematisch eine Schnittansicht einer Füllvorrichtung zum Abfüllen eines Füllprodukts in einen zu befüllenden Behälter in einer Füllproduktabfüllanlage; und
- Figur 2: schematisch eine Detail-Schnittansicht eines Füllventils einer Füllvorrichtung zum Abfüllen eines Füllprodukts in einen zu befüllenden Behälter in einer alternativen Ausführungsform.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

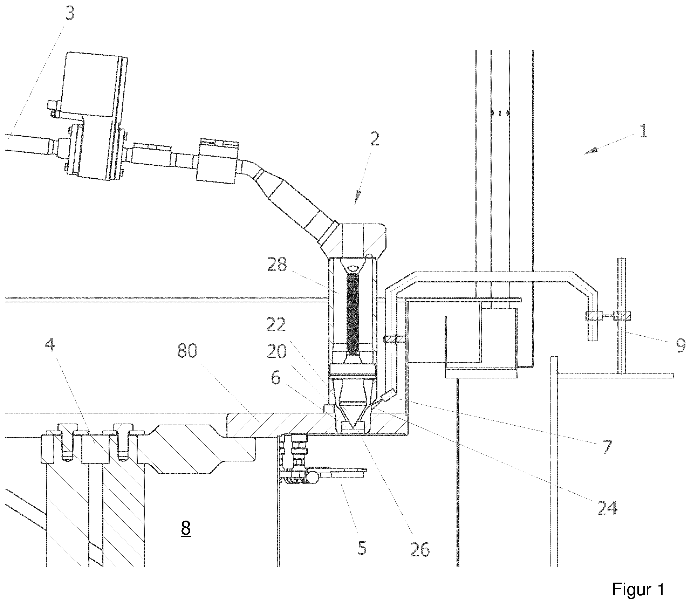

In Figur 1 ist schematisch eine Schnittansicht einer Füllvorrichtung 1 zum Abfüllen eines Füllprodukts in einen zu befüllenden Behälter in einer Füllproduktabfüllanlage gezeigt. Die Füllvorrichtung 1 ist in Rundläuferbauweise ausgebildet und weist ein drehbares Rundläuferkarussell 4 und einen gegenüber dem Rundläuferkarussell 4 statischen Teil 9 auf.

Ferner ist ein Reinraum 8 vorgesehen, in welchem das Abfüllen des Füllprodukts in den Behälter in einer kontrollierten Atmosphäre erfolgt. Der Reinraum 8 wird durch einen am Rundläuferkarussell 4 ausgebildeten Reinraumdeckel 80 und in den Figuren nicht weiter gezeigte Reinraumwände umschlossen beziehungsweise ausgebildet.

Am Umfang des Rundläuferkarussells 4 ist eine Vielzahl von Füllventilen 2 angeordnet, um in der Füllproduktabfüllanlage einen kontinuierlichen Strom an befüllten Behältern auf dem Rundläuferkarussell 4 produzieren zu können.

Die Füllventile 2 durchstoßen den Reinraumdeckel 80 derart, dass lediglich deren Produktauslass 26 und das Füllventilbodenstück 6 in den Reinraum 8 münden. Das Füllventilbodenstück 6 kann alternativ als Zentrierglocke ausgeführt sein, falls eine Befüllung von Behältern im angepressten Zustand durchgeführt wird.

Unterhalb der Füllventile 2 ist eine Vielzahl von Behälteraufnahmen 5 zum Aufnehmen der zu befüllenden Behälter angeordnet, wobei jeweils eine Behälteraufnahme 5 jeweils einem Füllventil 2 zugeordnet ist. Die Füllventile 2 sind jeweils über eine Produktleitung 3 mit einem nicht gezeigten Medienverteiler verbunden, über welchen sowohl das Füllprodukt aus einem Füllproduktreservoir als auch ein Behandlungsmedium in Form von Heißdampf aus einer Sterilisierungsvorrichtung zugeleitet wird.

Die Füllventile 2 weisen oberhalb ihres Produktauslasses 26 im Ventilinneren 28 einen Ventilkegel 22 auf, der in Richtung der Längsachse des Füllventils 2 beweglich angeordnet ist und der zum Schließen des Füllventils 2 in einen Ventilsitz 20 dichtend absenkbar ist. Zum Öffnen des Füllventils 2 wird der Ventilkegel 22 aus dem Ventilsitz 20 herausgehoben, so dass sich zwischen Ventilkegel 22 und Ventilsitz 20 ein Ringspalt ergibt, durch welchen hindurch das Füllprodukt oder ein Behandlungsmedium dann zum Produktauslass 26 gelangen und aus diesem austreten kann.

An dem stehenden Teil 9 ist ein Temperatursensor 7 angeordnet, der die Temperatur eines vorbeibewegten Füllventils 2 berührungslos bestimmen kann. Der berührungslose Temperatursensor 7 ist dabei an einer festen Position der Füllvorrichtung 1 vorgesehen. Er ist dabei derart positioniert, dass er im Bereich des Ventilkegels 20 an einer Außenseite 24 des Füllventils 2 die Temperatur des Füllventils 2 erfasst.

Der berührungslose Temperatursensor 7 ist dabei so eingerichtet, dass er die Temperatur jeweils eines der Mehrzahl der Füllventile 2, wenn sie sich an dem berührungslosen Temperatursensor 7 vorbeibewegen, bestimmen kann. Mit anderen Worten kann der berührungslose Temperatursensor 7 die Temperaturen der einzelnen Füllventile 2 voneinander differenzieren. Der berührungslose Temperatursensor 7 ist dadurch in der Lage, nacheinander die Temperatur eines jeden Füllventils 2, das mit dem Rundläuferkarussell 4 an ihm vorbeigeführt wird, zu erfassen.

Der berührungslose Temperatursensor 7 ist ferner mit einer nicht gezeigten Steuerungs- und/oder Regelungseinrichtung verbunden, mittels welcher die Behandlung, beispielsweise die Sterilisation der Füllventile 2, gesteuert und/oder geregelt sowie die Temperatur der Füllventile 2 überwacht werden kann.

Der berührungslose Temperatursensor 7 ist beispielsweise als Infrarotkamera ausgebildet. Alternativ kann er jedoch auch als Pyrometer oder in Form eines anderen berührungslosen Temperatursensors ausgebildet sein.

Zum Reinigen beziehungsweise thermischen Sterilisieren der Füllventile 2 wird über die Produktleitungen 3 Dampf zu den Füllventilen 2 geleitet. Um ein hinreichendes Bespülen der Kontaktflächen von Ventilkegel 22 und Ventilsitz 20 zu erzielen, werden die Füllventile 2 zu Anfang bevorzugt in einer offenen Stellung gehalten. Um den Verbrauch an Sterilisationsdampf möglichst gering zu halten, wird das Füllventil 2 im Anschluss teilweise oder vollständig geschlossen, so dass der Sterilisationsdampf im Wesentlichen im Ventilinneren 28 vorliegt. Alternativ kann auch eine Blende beziehungsweise Sterilisationskappe vor dem Produktauslass 22 angeordnet werden, um einen definierten Volumenstrom des Sterilisationsmediums zu erreichen.

Fällt der berührungslos erfasste Temperaturwert eines bestimmten Füllventils 2 unter einen vorgegebenen Temperaturwert, so wird das jeweilige Füllventil 2 geöffnet, das aufgrund des Temperaturabfalls möglicher Weise entstandene Kondensat aus dem Ventilinneren 28 ausgelassen und frischer Heißdampf zum Beheizen des Füllventils 2 zugeführt.

Als besonders vorteilhaft hat sich dabei herausgestellt, das Füllventil 2 alternierend zu öffnen und wieder zu schließen, wobei das Füllventil 2 anhand des erfassten Temperaturwerts, mithin temperaturabhängig, getaktet wird. Es wird folglich nur dann Behandlungsmedium über das geöffnete Füllventil ausgelassen, wenn es zum Aufheizen des Füllventils 2 und/oder Ablassen von Kondensat erforderlich ist. Auf diese Weise kann Behandlungsmedium eingespart werden.

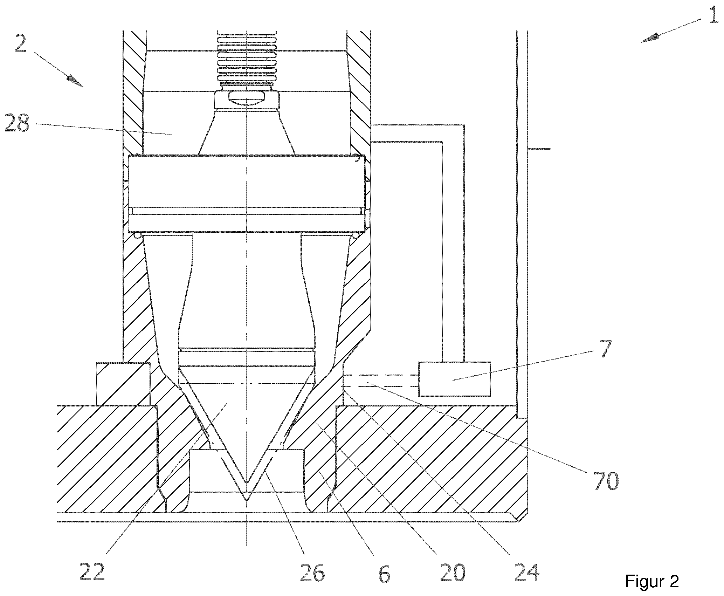

Figur 2 zeigt schematisch eine Detail-Schnittansicht eines Füllventils 2 einer Füllvorrichtung 1 zum Abfüllen eines Füllprodukts in einen zu befüllenden Behälter in einer alternativen Ausführungsform. Die hier gezeigte Füllvorrichtung 1 entspricht im Wesentlichen jener aus Figur 1. Im Gegensatz zur Füllvorrichtung 1 aus Figur 1 ist in der in Figur 2 gezeigten Ausführung jeweils ein berührungsloser Temperatursensor 7 an jedem der Mehrzahl von Füllventilen 2 angeordnet. Die Temperatursensoren 7 sind mithin am drehenden Teil, also dem Rundläuferkarussell 4, vorgesehen.

In der vorliegenden Ausführungsform sind die berührungslosen Temperatursensoren 7 als Pyrometer ausgebildet. Alternativ können aber auch andere berührungslose Temperatursensoren verwendet werden.

Das Füllventil 2 ist dabei derart ausgebildet, dass der berührungslose Temperatursensor 7 im Bereich des Ventilsitzes 20 an einer Außenseite 24 des Füllventils 2 die Temperatur des Füllventils 2 erfasst. Mit dem Bezugszeichen 70 ist der Messstrahl des Pyrometers angedeutet.

### Bezuqszeichenliste

- 1: Füllvorrichtung zum Abfüllen eines Füllprodukts in einen zu befüllenden Behälter
- 2: Füllventil
- 20: Ventilsitz
- 22: Ventilkegel
- 24: Außenseite
- 26: Füllproduktauslass
- 28: Ventilinneres
- 3: Produktleitung
- 4: Rundläuferkarussell
- 5: Behälteraufnahme
- 6: Füllventilbodenstück
- 7: berührungsloser Temperatursensor
- 70: Messstrahl
- 8: Reinraum
- 80: Reinraumdeckel
- 9: statischer Teil

## Patentansprüche

1. Füllvorrichtung (1) zum Abfüllen eines Füllprodukts in einen zu befüllenden Behälter in einer Füllproduktabfüllanlage, umfassend mindestens ein Füllventil (2) zum Dosieren des Füllprodukts in den zu befüllenden Behälter sowie eine Sterilisierungsvorrichtung zum Sterilisieren zumindest eines Teils des mindestens einen Füllventils (2), und
einen berührungslosen Temperatursensor (7) zum berührungslosen Erfassen der Temperatur des Füllventils (2),
**dadurch gekennzeichnet, dass**
ein Reinraum (8) vorgesehen ist und zumindest ein Füllproduktauslass (26) des Füllventils (2) in dem Reinraum (8) angeordnet ist, wobei der zumindest eine berührungslose Temperatursensor (7) außerhalb des Reinraums (8) so angeordnet ist, dass er die Temperatur des Füllventils (2) im Reinraum (8) erfassen kann.

2. Füllvorrichtung (1) gemäß Anspruch 1, **dadurch gekennzeichnet, dass** der zumindest eine berührungslose Temperatursensor (7) in Form eines Pyrometers und/oder einer Infrarotkamera ausgebildet ist.

3. Füllvorrichtung (1) gemäß einem der vorstehenden Ansprüche, **dadurch gekennzeichnet, dass** das mindestens eine Füllventil (2) an einer Transportvorrichtung, bevorzugt einem Rundläuferkarussell (4), angeordnet ist.

4. Füllvorrichtung (1) gemäß einem der vorstehenden Ansprüche **dadurch gekennzeichnet, dass** eine geringere Anzahl berührungsloser Temperatursensoren (7) als Füllventile (2), bevorzugt ein einziger berührungsloser Temperatursensor (7), zur berührungslosen Erfassung der Temperatur aller Füllventile (2) vorgesehen ist, wobei die Füllventile (2) relativ zu den berührungslosen Temperatursensoren (7) bewegbar sind, wobei bevorzugt die Temperatur des entsprechenden Füllventils (2) bei einem Vorbeibewegen des mindestens einen Füllventils (2) an dem zumindest einen berührungslosen Temperatursensor (7) erfasst werden kann.

5. Füllvorrichtung (1) gemäß einem der Ansprüche 1 bis 4, **dadurch gekennzeichnet, dass** eine Mehrzahl von Füllventilen (2) vorgesehen sind, wobei die Mehrzahl von Füllventilen (2) bevorzugt an einer Transportvorrichtung angeordnet ist, und jedem der Füllventile (2) jeweils ein berührungsloser Temperatursensor (7) zugeordnet ist.

6. Füllvorrichtung (1) gemäß einem der vorstehenden Ansprüche, **gekennzeichnet durch** eine mit dem zumindest einen berührungslosen Temperatursensor (7) verbundene Steuerungs- und/oder Regelungseinheit des Behandlungsvorgangs, welche die Behandlung des mindestens einen Füllventils (2) anhand des durch den zumindest einen berührungslosen Temperatursensor (7) erfassten Temperaturwerts des mindestens einen Füllventils (2) steuert/regelt.

7. Verfahren zum Sterilisieren zumindest eines Teils mindestens eines Füllventils (2) in einer Füllproduktabfüllanlage, umfassend das Beaufschlagen des zumindest einen Füllventils (2) mit einem Sterilisationsmedium, wobei
die Temperatur des zumindest einen Füllventils (2) berührungslos erfasst wird,
**dadurch gekennzeichnet, dass**
ein Reinraum (8) vorgesehen ist und zumindest ein Füllproduktauslass (26) des Füllventils (2) in dem Reinraum (8) angeordnet ist und die Temperatur des mindestens einen Füllventils (2) außerhalb des Reinraums (8) erfasst wird.

8. Verfahren gemäß Anspruch 7, **dadurch gekennzeichnet, dass** die Sterilisierung des mindestens einen Füllventils (2) anhand des berührungslos erfassten Temperaturwerts des mindestens einen Füllventils (2) gesteuert/geregelt wird.

9. Verfahren gemäß einem der Ansprüche 7 oder 9, **dadurch gekennzeichnet, dass** das zumindest eine Füllventil (2) während der Sterilisierung geöffnet wird, wenn der berührungslos erfasste Temperaturwert des Füllventils (2) unter einen vorgegebenen Temperaturwert fällt.

## Claims

1. Filling device (1) for filling a filling product into a container to be filled in a filling product filling installation, comprising at least one filling valve (2) for metering the filling product into the container to be filled, as well as a sterilising apparatus for sterilising at least a part of the at least one filling valve (2), and
a contactless temperature sensor (7) for the contactless detecting of the temperature of the filling valve (2),
**characterised in that**
a clean room (8) is provided and at least one filling product outlet (26) of the filling valve (2) is arranged in the clean room (8), wherein the at least one contactless temperature sensor (7) is arranged outside the clean room (8) such that it is able to detect the temperature of the filling valve (2) in the clean room (8).

2. Filling device (1) according to claim 1, **characterised in that** the at least one contactless temperature sensor (7) is provided in the form of a pyrometer and/or an infrared camera.

3. Filling device (1) according to any of the preceding claims, **characterised in that** the at least one filling valve (2) is arranged on a transport device, preferably a revolving carousel (4).

4. Filling device (1) according to any of the preceding claims, **characterised in that** a lower number of contactless temperature sensors (7) than of filling valves (2) is provided, preferably one single contactless temperature sensor (7), for the contactless detecting of the temperature of all filling valves (2), wherein the filling valves (2) are movable in relation to the contactless temperature sensors (7), wherein preferably the temperature of the corresponding filling valve (2) can be detected by moving the at least one filling valve (2) past the at least one contactless temperature sensor (7).

5. Filling device (1) according to any of claims 1 to 4, **characterised in that** a plurality of filling valves (2) is provided, wherein the plurality of filling valves (2) is preferably arranged on a transport apparatus, and a contactless temperature sensor (7) is assigned in each case to one of the filling valves (2).

6. Filling device (1) according to any of the preceding claims, **characterised by** a controlling and/or regulating unit, connected to the at least one contactless temperature sensor (7), of the treatment procedure, which controls the treatment of the at least one filling valve (2) on the basis of the temperature value of the at least one filling valve (2) detected by the at least one contactless temperature sensor (7).

7. Method for sterilising at least a part of at least one filling valve (2) in a filling product filling installation, comprising applying a sterilisation medium to the at least one filling valve (2), wherein
the temperature of the at least one filling valve (2) is detected contactlessly,
**characterised in that**
a clean room (8) is provided and at least one filling product outlet (26) of the filling valve (2) is arranged in the clean room (8) and the temperature of the at least one filling valve (2) is detected outside the clean room (8).

8. Method according to claim 7, **characterised in that** the sterilisation of the at least one filling valve (2) is controlled/regulated on the basis of the contactlessly detected temperature value of the at least one filling valve (2).

9. Method according to any of claims 7 or 8, **characterised in that** the at least one filling valve (2) is opened during the sterilisation when the contactlessly detected temperature value of the filling valve (2) falls below a predetermined temperature value.

## Revendications

1. Dispositif de remplissage (1) destiné à remplir un contenant à remplir avec un produit de remplissage dans une installation de remplissage en produit de remplissage, comprenant au moins une soupape de remplissage (2) pour doser le produit de remplissage dans le contenant à remplir et un dispositif de stérilisation pour stériliser à au moins une partie de la au moins une soupape de remplissage (2), et
un capteur de température sans contact (7) destiné à détecter sans contact la température de la soupape de remplissage (2),
**caractérisé en ce que**
un espace propre (8) est prévu et au moins une sortie de produit de remplissage (26) de la soupape de remplissage (2) est agencée dans l'espace propre (8), dans lequel le au moins un capteur de température sans contact (7) est agencé à l'extérieur de l'espace propre (8) de manière à ce qu'il puisse détecter la température de la soupape de remplissage (2) dans l'espace propre (8).

2. Dispositif de remplissage (1) selon la revendication 1, **caractérisé en ce que** le au moins un capteur de température sans contact (7) est conçu sous la forme d'un pyromètre et/ou d'une caméra infrarouge.

3. Dispositif de remplissage (1) selon l'une quelconque des revendications précédentes, **caractérisé en ce que** la au moins une soupape de remplissage (2) est agencée au niveau d'un dispositif de transport, de préférence un carrousel rotatif (4).

4. Dispositif de remplissage (1) selon l'une quelconque des revendications précédentes, **caractérisé en ce qu'un** nombre plus restreint de capteurs de température sans contact (7) que de soupapes de remplissage (2) est prévu, de préférence un seul capteur de température sans contact (7), pour détecter sans contact la température de toutes les soupapes de remplissage (2), dans lequel les soupapes de remplissage (2) sont mobiles par rapport aux capteurs de température sans contact (7), dans lequel de préférence la température de la soupape de remplissage correspondante (2) peut être détectée lors du passage de la au moins une soupape de remplissage (2) devant le au moins un capteur de température sans contact (7).

5. Dispositif de remplissage (1) selon l'une quelconque des revendications 1 à 4, **caractérisé en ce qu'**une pluralité de soupapes de remplissage (2) sont prévues, dans lequel la pluralité de soupapes de remplissage (2) est de préférence agencée sur un dispositif de transport, et chacune des soupapes de remplissage (2) est associée à un capteur de température sans contact (7).

6. Dispositif de remplissage (1) selon l'une quelconque des revendications précédentes, **caractérisé par** une unité de commande et/ou de régulation du processus de traitement reliée au au moins un capteur de température sans contact (7), qui commande/régule le traitement de la au moins une soupape de remplissage (2) à l'aide de la valeur de température de la au moins une soupape de remplissage (2) détectée par le au moins un capteur de température sans contact (7).

7. Procédé de stérilisation d'au moins une partie d'au moins une soupape de remplissage (2) dans une installation de remplissage en produit de remplissage, comprenant l'alimentation de la au moins une soupape de remplissage (2) avec un milieu de stérilisation, dans lequel
la température de la au moins une soupape de remplissage (2) est détectée sans contact,
**caractérisé en ce que**
un espace propre (8) est prévu et au moins une sortie de produit de remplissage (26) de la soupape de remplissage (2) est agencée dans l'espace propre (8) et la température de la au moins une soupape de remplissage (2) est détectée à l'extérieur de l'espace propre (8).

8. Procédé selon la revendication 7, **caractérisé en ce que** la stérilisation de la au moins une soupape de remplissage (2) est commandée/régulée à l'aide de la valeur de température de la au moins une soupape de remplissage (2) détectée sans contact.

9. Procédé selon l'une quelconque des revendications 7 ou 9, **caractérisé en ce que** la au moins une soupape de remplissage (2) est ouverte pendant la stérilisation si la valeur de température de la soupape de remplissage (2) détectée sans contact descend en dessous d'une valeur de température prédéterminée.
